# EUROPÄISCHE PATENTSCHRIFT

(11) **EP 0 644 776 B1**
(45) Veröffentlichungstag und Bekanntmachung des Hinweises auf die Patenterteilung: **02.12.1998**
(21) Anmeldenummer: 93911501.0
(22) Anmeldetag: 24.04.1993
(51) Int. Cl.: A61K 49/00, G01S 15/89

(54) **VERWENDUNG VON MIKROKAPSELN ALS KONTRASTMITTEL FÜR DIE FARBDOPPLER-SONOGRAPHIE**
USE OF MICROCAPSULES AS CONTRASTING AGENTS IN COLOUR DOPPLER SONOGRAPHY
UTILISATION DE MICROCAPSULES COMME AGENTS DE CONTRASTE POUR LA SONOGRAPHIE DOPPLER EN COULEURS

(30) Priorität: 13.06.1992 DE 4219724
(43) Veröffentlichungstag der Anmeldung: 29.03.1995
(73) Patentinhaber: SCHERING AKTIENGESELLSCHAFT, 13353 Berlin (DE)
(72) Erfinder: REINHARDT, Michael, Dr., D-10999 Berlin (DE); FRITZSCH, Thomas, Dr., D-12169 Berlin (DE); HELDMANN, Dieter, Dr., D-10555 Berlin (DE); SIEGERT, Joachim, Dr., D-12157 Berlin (DE)
(86) Internationale Anmeldenummer: EP9300991
(87) Internationale Veröffentlichungsnummer: WO9325241

(56) Entgegenhaltungen:
- EP-A- 0 327 490
- EP-A- 0 484 181
- WO-A-91/15999
- STN FILE SUPPLIER & FILE MEDLINE AN=89333475 (KARLSRUHE)
- STN FILE SUPPLIER & FILE MEDLINE AN=9317479
- STN FILE SUPPLIER & FILE MEDLINE AN=91297395
- REV. PRAT. Bd. 40, Nr. 30, 21. Dezember 1990, Seiten 2779 - 2784 MC. MALLERGUE 'COLOR DOPPLER ECHOCARDIOGRAPHY.'

## Beschreibung

Die Patentanmeldung betrifft den in den Patentansprüchen gekennzeichneten Gegenstand, d.h. die Verwendung von Mikrokapseln zur Kontrastierung des retikuloendothelialen Systems, des Myocards und/oder des Gastrointestinaltrakts und anderen Körperhöhlen mittels der Farbdoppler-Sonographie.

Im Bereich der Ultraschall-Diagnostik werden je nach diagnostischer Fragestellung verschiedene Aufnahmetechniken, wie z. B. die M-Mode-, B-Mode-, CW-Doppler-, PW-Doppler- sowie die FarbdopplerTechnik verwendet. M- und B-Mode-Techniken basieren auf dem Prinzip, daß Ultraschallwellen im Megahertz-Bereich (oberhalb 2 Mega-Hertz mit Wellenlängen zwischen 1 und 0,2 nm) an Grenzflächen von Medien mit unterschiedlicher akustischer Dichte reflektiert werden. Die dadurch entstehenden Echos werden verstärkt und sichtbar gemacht. Die Methode ist prinzipiell zur Darstellung verschiedener Organe aber auch zur Abbildung sich bewegender Strukturen, wie z.B. Herzklappen, geeignet.

Im Gegensatz dazu werden die Dopplertechniken ausschließlich zur Untersuchung sich bewegender Strukturen, z. B. zur Quantifizierung der Geschwindigkeit von Blutflüssen, verwendet. So basiert das Messprinzip dieser Techniken darauf, daß das im Ultraschallfeld an bewegten Körpern (z. B. rote Blutzellen) reflektierte Signal einen Frequenzshift (den sogenannten Doppler-Shift) erfährt. Dieser Frequenzshift wird bei der Farbdopplersonographie in Farbsignale umgewandelt, so daß bewegte Strukturen farbig codiert, starre Strukturen jedoch als Grauwert abgebildet werden [R. Omoto (1987) Real-time two-dimensional Doppler echocardiography, 2nd edn. Lea & Febiger, Philadelphia].

Sowohl den DopplerTechniken als auch den anderen bekannten Techniken wie z.B. B- und M-Mode ist gemein, daß die Kontrastierung und damit die diagnostische Aussage verbessert wird, wenn feine Gasbläschen an den Untersuchungsort gebracht werden (siehe Gramiak, Invest. Radiol 3 (1968) 356/366).
Diese Gasbläschen können auf die verschiedenste Art und Weise hergestellt werden; z.B. durch Schütteln oder andere Agitation von physiologisch verträglichen Lösungen (EP 0 077 752; Roelandt, J. Ultrasound. Med. Biol. 8: 471-492, 1982).

In der EP 0 052 575 werden Gasbläschen erzeugt, indem eine feste kristalline Substanz, wie beispielsweise Galaktose, in einer Trägerflüssigkeit suspendiert wird. Die Gasbläschen entstehen dabei aus in Hohlräumen eingeschlossenem, bzw. aus an der Kristalloberfläche adsorbiertem Gas.

Die EP 0 122 624 beschreibt ein ähnliches Kontrastmittel auf der Basis einer nicht grenzflächenaktiven Substanz wie z. B. Galaktose, der eine grenzflächenaktive Substanz wie z.B. Magnesiumstearat beigemengt wird, was zu einer Stabilisierung der Gasbläschen führt. Damit wurde auch die Kontrastierung der linken Herzseite, sowie verschiedener Organe wie Leber, Milz und Niere im 2D-Echo-Bild oder im M-Mode-Echo-Bild möglich.

Die DE 38 03 972 offenbart gas- bzw. flüssigkeitsgefüllte Mikrokapseln auf der Basis von bioabbaubaren Polymeren wie z.B. Polycyanacrylaten oder α-,β-,γ-Hydroxycarbonsäuren.
Ähnliche Mikrokapseln werden in der Europäischen Patentanmeldung EP 0 441 468 beschrieben. Im Gegensatz zu den in der DE 38 03 972 offenbarten Partikeln ist die Partikelhülle in diesem Falle aus Polyaldehyden aufgebaut.
Die EP 0 224 934 und die US 4,276,885 beschreiben gasgefüllte Mikrokapseln auf der Basis von Proteinen bzw. Albuminen (EP) bzw. auf der Basis von Gelatine (US). Den Partikeln der DE 38 03 972 und der EP 0 441 468 ist gemein, daß sie ausreichend stabil und genügend Klein (<10µm) sind, um kapillargängig zu sein und intrazellulär im Retikulo-endothelialen System (wie z. B. Leber, Lymphknoten und Milz) aufgenommen zu werden. Jedoch ist die Kontrastierung bei Verwendung der B-Mode bzw. M-Mode-Technik nicht in allen Fällen zufriedenstellend, z.B. ist Abgrenzung von gesundem Gewebe (z. B. Leber, Milz, Lymphknoten) gegenüber Tumorgewebe, das nur wenige zum retikuloendothetialen System gehörenden Zellen enthält oft nicht möglich.

Daneben bereitet die Darstellung des Gastrointestinaltrakts sowie der Perfusion des Myocards Schwierigkeiten.

Aufgabe der Erfindung ist es, die Kontrastierung der genannten Bereiche zu verbessern.

Es wurde nun festgestellt, daß bei Verabreichung bestimmter Ultraschall-Kontrastmittel überraschend auch in Körperzonen, in denen keine Partikelbewegung stattfindet, das heißt im RES (Leber, Milz und Lymphknoten), im Gastrointestinaltrakt, sowie im Myocard, entgegen der theoretischen Voraussage auch bei Anwendung der herkömmlichen Farbdoppler-Sonographie eine Kontrastierung zu erreichen ist.

Unter "keiner Partikelbewegung" werden Bewegungsgeschwindigkeiten verstanden, die Null bzw. deutlich geringer sind als die bei Perfusion der Blutgefäße auftretenden Strömungsgeschwindigkeiten. Solche Geschwindigkeiten werden als nicht ausreichend für eine Erzeugung eines Farbdoppler-Signals angesehen.

Somit bezieht sich die Erfindung auf die Verwendung von gas- oder flüssigkeitsgefüllten Mikrokapseln zur Herstellung eines Ultraschallkontrastmittels zur Kontrastierung von Körperregionen in denen im wesentlichen keine Bewegung der Mikrokapseln zu beobachten ist mittels Farbdoppler-Sonographie. bestehenden Kontrastmitteln zur Kontrastierung von Körperregionen, in denen keine Bewegung der Mikrokapseln zu verzeichnen ist, wobei die Aufnahme dennoch mit der Farbdoppler-Technik erfolgt.

Es war weiterhin überraschend, daß die so erzielte Kontrastierung besser ist, als die mit den konventionellen Methoden (B-Mode, M-Mode) erreichbare. Dieses führt zu einer deutlichen Verbesserung des diagnostischen Aussagewertes.

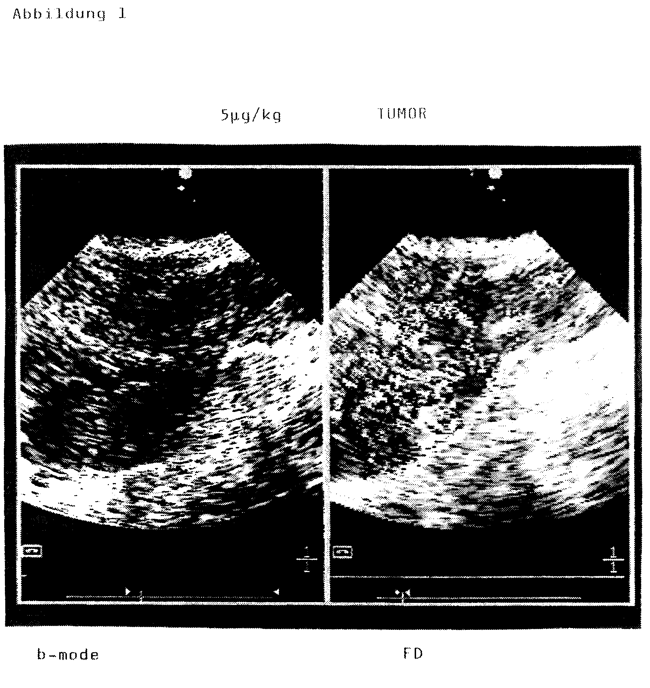

So werden z. B. bei der Untersuchung von Lebergewebe mittels der Farbdoppler-Sonographie, nach intravenöser Injektion des Kontrastmittels, Tumorareale (die bekanntermaßen keine oder nur wenige zur Aufnahme der Partikel befähigten Zellen enthalten) in den gewohnten Grauwerten abgebildet, gesundes Gewebe hingegen überraschenderweise farbig (siehe Abb. 1/rechte Bildhälfte). Ein Vergleichsphoto im B-Mode, das unter sonst identischen Bedingungen aufgenommen wurde, (siehe Abb. 1/linke Bildhälfte) bestätigt den deutlich verbesserten diagnostischen Aussagewert der Farbdoppler Aufnahme. Tumorbezirke sind nicht mehr von den gesunden Bereichen abzugenzen.

Nach oraler Applikation ist eine Markierung des Magen-Kanals möglich, die z.B. eine Abgrenzung intestinaler Strukturen von anderen abdominellen Organen ermöglicht.

Die Darstellung des Gelenkspaltes gelingt nach Verabreichung des Kontrastmittels und unter Nutzung der Dopplertechniken ebenso wie die Abbildung der Tuben und des Cavum Uteri.

Geeignete Mikrokapseln bestehen in der Regel aus einer dünnwandigen Hülle, z. B. einem synthetischen bioabbaubaren polymeren Material und einem Kern, der aus einem Gas oder einer niedrig siedenden Flüssigkeit besteht.
Als geeignete Gase kommen z. B. Luft, Stickstoff, Sauerstoff, gasförmige Kohlenwasserstoffe, Edelgase und Kohlendioxid in Frage. Als niedrig siedende Flüssigkeiten eignen sich 1,1-Dichlorethylen, 2-Methyl-2-buten, 2-Methyl-1,3-Butadien, 2-Butin, 2-Methyl-1-Buten, Dibrom-difluormethan, Furan, 3-Methyl-1-Buten, Isopentan, Diethylether, 3,3-Dimethyl-1-Butin, Propylenoxid, Bromethan, Pentan, Cyclopentan, 2,3-Pentadien, Cyclopentan und deren Gemische.
Als Hüllmaterial eignet sich z. B. bioabbaubares synthetisches Material wie z. B. Polyaldehyde, die gewünschtenfalls zur Copolymerisation fähige Zusätze und/oder Crosslinker, gegebenenfalls Tenside oder Tensidgemische, Kopplungsagentien sowie gegebenenfalls über diese Kopplungsagenzienen gebundene Bio- oder Makromoleküle enthalten, Polycyanacrylat oder Polyester von α-, β- oder γ-Hydroxycarbonsäuren.

Geeignete Hüllmaterialien für gasgefüllte Miktokapseln sind Gelatine oder Proteine wie z. B. teilweise denaturiertes Albumin oder humanes Serumalbumin.

Die Form der Partikel kann beliebig geartet sein (z. B. spherisch, ellipsoid usw.), die Wandstärke der Partikel ist vorteilhafterweise so dünn wie möglich zu wählen, vorzugsweise <200 nm.
Die Partikelgröße ist durch die jeweilige Anwendung limitiert, bei Untersuchungen des RES sowie des Myocards sollte sie zwischen 1 bis 10 µm liegen, um Kapillargängigkeit zu gewährleisten, bei Untersuchungen von Körperhöhlen sowie des Gastrointestinaltrakts sind auch Partikel bis zu einer Größe von 100µm geeignet.

Derartig erfindungsgemäß zu verwendende Mittel werden in den Patentschrifien DE 38 03 972, in der Europäischen Patentanmeldung 0 441 468, im Europäischen Patent 0 224 934 sowie in der US-Patentschrift 4,276,885 beschrieben.

Erfindungsgemäß bevorzugt sind gasgefüllte Mikrokapseln auf der Basis von Polycyanacrylaten.

Ein neues bevorzugtes Herstellungsverfahren dieser Partikel besteht darin, daß monomeres Cyanacrylat in einer sauren, mit einem Gas oder Gasgemischen gesättigten, wässrigen Lösung, die gegebenenfalls mindestens eine oberflächenaktive Substanz enthält, mit einem Rotor-Stator-Mischer dispergiert wird, die nach 5 Minuten bis 3 Stunden Dispergierung erhaltenen Partikel abgetrennt werden, gegebenenfalls mit Wasser gewaschen werden, anschließend in einem pharmazeutisch akzeptablen Suspensionsmedium aufgenommen und gefriergetrocknet werden, wobei die Suspension vorteilhafterweise während des Einfrierens kräftig bewegt wird. Vorzugsweise wird als Cyanacrylat der Butylester, als Gas Luft, Stickstoff, Sauerstoff, Edelgase oder Kohlendioxid eingesetzt. Anstelle des Rotor-Stator-Mischers können auch vergleichbare Geräte (wie z.B. ein Dissolver-Rührer) verwendet werden, die ein kräftiges Dispergieren der Mischung erlauben. Als oberflächenaktive Substanz wird (werden) bevorzugt (eine) Substanz(en) aus der Gruppe Polysorbate, Octyl- oder Nonylphenole, Macrogol-glycerolester oder Cetomacrogole oder Poloxamere ® oder deren Gemische, verwendet. Der pH-Wert der wässrigen gasgesättigten Lösung liegt vorzugsweise zwischen 1,8 und 4,5, zur Einstellung des pH-Wertes eignen sich insbesondere Salz- und Phosphorsäure. Die Abtrennung der Partikel erfolgt mittels Zentrifugation oder Flotation. Als Suspensionsmedium eignet sich Wasser für Injektionszwecke gegebenenfalls mit einem Zusatz von Kochsalz und/oder Glucose und/oder Mannitol und/oder Lactose, das gegebenenfalls zusätzlich noch eine oberflächenaktive Substanz, wie z.B. aus der Gruppe der Polysorbate, Octyl- oder Nonylphenole, Macrogol-glycerolester oder Cetomacrogole oder Substanzen aus der Gruppe der Poloxamere ®oder deren Gemische und/oder einen mehrwertigen Alkohol enthält.

Ein neues bevorzugtes Herstellungsverfahren von Partikeln auf der Basis von Polyestern besteht darin, daß ein Polyester einer α-,β- oder γ - Hydroxycarbonsäure, gegebenenfalls gemeinsam mit einem wasser-dispergierbaren Emulgator, in einem gesundheitlich unbedenklichen Lösungsmittel gelöst wird und diese Lösung unter Dispergierung mit einem Dissolver Rührer oder einem Schallstab zu einer gashaltigen Flüssigkeit gegeben wird, die sofern der Emulgator nicht bereits gemeinsam mit dem Polyester zugesetzt wurde, einen wasser-dispergierbaren Emulgator enthält, die nach 30 Minuten bis 2 Stunden Dispergierung erhaltenen Partikel abgetrennt werden, gegebenenfalls mit Wasser gewaschen werden, anschließend in einem pharmazeutisch akzeptablen Suspensionsmedium aufgenommen und gefriergetrocknet werden. Erfindungsgemäß bevorzugt sind Polymere der Milchsäure oder der Glycolsäure sowie deren Mischpolymerisate. Als unbedenkliches Lösungsmittel wird vorzugsweise erhitzter Ethylalkohol verwendet. Als gashaltige Flüssigkeit wird vorzugsweise Wasser oder Glycerol 87 % verwendet, die bevorzugten Gase sind Luft, Stickstoff, Sauerstoff, Edelgase oder Kohlendioxid. Als wasserdispergierbarer Emulgator sind Phosphatidycholin oder Sucrose-Palmitat-Stearat 15 sowie deren Gemische zu nennen. Als pharmazeutisch akzeptables Suspensionsmedium eignen sich dieselben Medien wie im Falle der Partikel auf der Basis von Polycyanacrylat.

Zur Erreichung der erforderlichen Konzentration von 10⁵-10⁷ Partikel/cm³ Target-Organ müssen die folgenden Partikelkonzentrationen verabreicht werden:
10⁸-10¹⁰ Partikel/kg Körpergewicht bei Untersuchungen des Gastrointestinaltrakts,
10⁷-10⁹ Partikel/kg Körpergewicht bei Untersuchungen des Lymphsystems,
10⁷-10⁹ Partikel/kg Körpergewicht bei Untersuchungen des RE-Systems und des Myocards.
Die Konzentration des verabreichten Kontrastmittels kann hierbei im Bereich bis zu 2x10¹⁰ Partikel/ml liegen.
Unterschiedlich sind auch die Zeiträume, die im Anschluß an die Applikation bis zum Beginn der Untersuchung verstreichen müssen, um das gewünschte Ergebnis zu erzielen. Erfindungsgemäß bevorzugte Zeitintervalle sind:
10 Sekunden bei Untersuchungen des Myocards,
1-60 Minuten p.i. bei Untersuchungen des RE-Systems,
5-10 Minuten p.i. bei Untersuchungen des Lymphsystems,
5-60 Minuten bei Magen-Darm Untersuchungen.
Im Falle von Körperhöhlen und Blasenuntersuchungen kann mit der Untersuchung unmittelbar nach dem Einbringen des Kontrastmittels begonnen werden.

Die Erfindung wird durch die nachfolgenden Beispiele erläutert:

### Beispiel 1

0,4 ml Cyanacrylsäurebutylester werden in 60 ml Salzsäure von pH 2,0 die 1 % Poloxamer 407 enthält, mit einem Rotor-Stator-Mischer 5 Minuten lang dispergiert. Die Mikropartikel mit einer durchschnittlichen Größe von 2 µm werden abzentrifugiert und in 300 ml einer wässrigen Lösung von 1 % Poloxamer und 5 % Glucose aufgenommen. Die Dichtebestimmung ergibt ein spezifisches Gewicht von 0,2 g/cm³.

### Beispiel 2

Es wird wie in Beispiel 1 verfahren, wobei die Salzsäure einen pH-Wert von 2,5 aufweist und Poloxamer 407 durch Octoxynol-9 ersetzt wird. Die Mikropartikel besitzen eine durchschnittliche Größe von etwa 0,9 µm und ein spezifisches Gewicht von 0,2 g/ cm³. Sie werden in 300 ml 5 %iger Mannitol-Lösung, die 0,1 % Polysorbat 20 enthält, aufgenommen.

### Beispiel 3

Es wird wie in Beispiel 1 verfahren, wobei die Salzsäure einen pH-Wert von 3,0 aufweist und Poloxamer 407 durch Cetomacrogol 1200 ersetzt wird. Die Durchschnittsgröße der Mikropartikel beträgt 1,5 µm, ihr spezifisches Gewicht 0,3 g/cm³. Sie werden in 300 ml 5 %iger Mannitol-Lösung, die 0,1 % Cetomacrogol 1200 und 5 % Povidone enthält, aufgenommen.

### Beispiel 4

Es wird wie in Beispiel 1 verfahren, wobei Poloxamer 407 durch 5 % Polysorbat 40 ersetzt wird. Die Durchschnittsgröße der Mikropartikel beträgt 1,0 µm, ihr spezifisches Gewicht 0,4 g/cm³. Sie werden in 300 ml 5 %iger Mannitol-Lösung, die 1 % Macrogolglycerolhydroxystearat enthält, aufgenommen.

### Beispiel 5

Es wird wie in Beispiel 1 verfahren, wobei Poloxamer 407 durch 5 % Macrogolglycerolhydroxystearat ersetzt wird. Die Partikel sind durchschnittlich 0,9 µm groß und haben ein spezifisches Gewicht von 0,3 g/cm³. Sie werden in 300 ml 5 %iger Mannitol-Lösung, die 1 % Macrogolglycerolhydroxystearat und 10 % Propylenglykol enthält, aufgenommen.

### Beispiel 6

Es wird wie in Beispiel 1 verfahren, wobei Cyanacrylsäurebutylester durch Cyanacrylsäureethylester ersetzt wird. Die Mikropartikel haben eine durchschnittliche Größe von 1,5 µm und ein spezifisches Gewicht von 0,2/cm³. Sie werden in 300 ml einer wässrigen Lösung von 1 % Poloxamer 407 und 5 % Glucose aufgenommen.

### Beispiel 7

Es wird wie in Beispiel 1 verfahren, wobei Cyanacrylsäurebutylester durch Cyanacrylsäureisopropylester ersetzt wird. Die Mikropartikel haben eine durchschnittliche Größe von 1,3 µm, und ein spezifisches Gewicht von 0,2 g/cm³. Sie werden in 300 ml einer wässrigen Lösung von 1 % Poloxamer 407 und 5 % Mannitol und 10 % Propylenglykol aufgenommen.

### Beispiel 8

3 ml Cyanacrylsäurebutylester werden in 300 ml Salzsäure von pH 2,0, die 1 % Poloxamer 407 enthält, mit einem Dissolver-Mischer 120 Minuten lang dispergiert. Die Mikropartikel mit einer durchschnittliche Größe von 2µm und einem spezifischen Gewicht von 0,1 g/cm³ werden durch Flotation abgetrennt und in 5 l einer 5 %igen Mannitol-Lösung, die 1 % Poloxamer 407 und 10 % Propylenglykol enthält, aufgenommen.

### Beispiel 9

Es wird wie in Beispiel 8 verfahren, wobei Poloxamer 407 durch Octoxynol-9 ersetzt und der pH-Wert auf 2,5 eingestellt wird. Die Durchschnittsgröße der Mikropartikel beträgt 0,8 µm, ihr spezifisches Gewicht 0,15 g/cm³. Sie werden in 5 l einer 0,9 %igen Kochsalzlösung, die 0,1 % Cetomacrogol 1200 enthält, aufgenommen.

### Beispiel 10

Es wird wie in Beispiel 8 verfahren, wobei Poloxamer 407 durch Cetomacrogol 1200 ersetzt wird. Die Durchschnittsgröße der Mirkopartikel beträgt 1,8 µm, ihr spezifisches Gewicht 0,4 g/cm³. Sie werden in 5 l einer 5 %igen Glucoselösung, die Cetomacrogol 1200 enthält, aufgenommen.

### Beispiel 11

Es wird wie in Beispiel 8 verfahren, wobei Poloxamer 407 durch 5 % Polysorbat 60 ersetzt wird. Die Durchschnittsgröße der Mirkopartikel beträgt 1,0 µm, ihr spezifisches Gewicht 0,4 g/cm³. Sie werden in 5 l einer 5 %igen Mannit-Lösung, die 1 % Poloxamer 407 und 10 % Propylenglykol enthält, aufgenommen.

### Beispiel 12

Die in Beispiel 8, 9, 10 oder 11 hergestellten Partikel werden statt in dort angegebenen Lösungen in je 5 l einer 5 %igen Mannitol-Lösung, die 0,1 % Cetomacrogol 1200 und 5 % Povidone enthält, aufgenommen in 15 ml - Portionen unter kräftigem Schütteln eingefroren und gefriergetrocknet. Vor Anwendung wird das Lyophilisat mit Wasser für Injektionszwecke resuspendiert und gegebenenfalls filtriert.

### Beispiel 12a

Die in Beispiel 8, 9, 10 oder 11 hergestellten Partikel werden statt in dort angegebenen Lösungen in je 5 l einer 10 %igen Lactose-Lösung, die 0,1 % Cetomacrogol 1200 enthält, aufgenommen, in 15 ml - Portionen unter kräftigem Schütteln eingefroren und gefriergetrocknet. Vor der Anwendung wird das Lyophilisat mit Wasser für Injektionszwecke resuspendiert und gegebenenfalls filtriert.

### Beispiel 13

1,0 g hydriertes Sojalecithin werden in 200 ml Glycerol mit einem Dissolver-Mischer dispergiert. Zu der Dispersion werden nach 60 Minuten 2,0 g in 10 ml siedendem Ethanol gelöstes Poly-L-Lactid (mittleres Molekulargewicht 1100) zugetropft. Es wir 60 Minuten lang weiter dispergiert. Die entstandenen Mirkopartikel werden bei 1000 Upm zentrifugiert, der Überstand in 50 ml Wasser aufgenommen, erneut zentrifugiert (1000 Upm), der Überstand wird in 5 %iger Mannitol-Lösung aufgenommen. Diese Suspension wird in 10 ml Portionen aufgeteilt und gefriergetrocknet. Das Lyophilisat wird vor Anwendung mit Wasser für Injektionszwecke resuspendiert.

### Beispiel 14

1,0 g Sucrose-Palmitat-Stearat (HLB 15) werden in 200 ml Glycerol mit einem Dissover-Mischer dispergiert. In die Dispersion wird nach 30 Minuten 1,0 g in 10 ml siedendem Ethanol gelöstes Poly-L-Lactid (mittleres Molekulargewicht 1100) zugetropft. Es wird 60 Minuten weiterdispergiert. Die entstandenen Mikropartikel haben eine durchschnittliche Größe von 2 µm. Sie werden bei 1000 Upm 30 Minuten lang zetrifugiert, der Überstand wid in 50 ml Wasser aufgenommen, erneut zentrifugiert (1000 Upm), der Überstand wird in 50 ml einer 5 %igen Mannitol-Lösung aufgenommen. Diese Suspension wird in 10 ml-Portionen aufgeteilt und gefriergetrocknet. Das Lyophilisat wird vor Anwendung mit Wasser für Injektionszwecke resuspendiert.

### Beispiel 15

Einem Hund (11 kg Inhalationsnarkose) werden nach Beispiel 8 hergestellte Mikropartikel (250 µg/ml) in einer Dosis von 300 µg/kg Körpergewicht peripher - venös mit einer Geschwindigkeit von 0,1 ml/s injiziert. Nach 10 Minuten stellt sich die Leber bei der Farbdoppleruntersuchung über einen zur Untersuchung ausreichend langen Zeitraum homogen farbcodiert dar.

### Beispiel 16

Man wiederholt Beispiel 15 mit den in den Beispielen 1 bis 7 oder 9 bis 14 hergestellten Partikeln. Auch hierbei stellt sich die Leber homogen farbcodiert dar.

### Beispiel 17

Einem Kaninchen (3,5 kg, Inhalationsnarkose) werden nach Beispiel 2 hergestellte Mikropartikel in einer Dosis von 5 µg/kg Körpergewicht peripher - venös injiziert. Nach 10 Minuten stellt sich die Leber bei der Farbdoppleruntersuchung farbcodiert dar, wohingegen Tumorbezirke in den gewohnten Grauwerten erscheinen (siehe Abbildung 1/rechte Bildhälfte). Zum Vergleich wurde auch eine Aufnahme im B-mode unter sonst identischen Bedingungen angefertigt (siehe Abbildung 1/linke Bildhälfte). In dieser Aufnahme ist eine gleichmäßige Verschattung der Leber zu erkennen, eine Abgrenzung der Tumorareale ist nicht mehr möglich.

### Beispiel 18

4 g Terephthalsäuredichlorid werden in 20 ml Cyclohexan gelöst und in 500 ml 3 %iger Natriumcarbonatlösung, die 1 % Poloxamer 407 enthält, mit einem Propellerrührer emulgiert. Nach Zusatz von 600 mg L-Lysin, in 50 ml 1 %iger Poloxamer 407 - Lösung gelöst, werden die Kapseln mit einer durchschnittlichen Größe von 30 µm abzentrifugiert, in einer zur Injektion geeigneten Flüssigkeit resuspendiert, eingefroren und gefriergetrocknet. Nach Resuspendierung dieser Zubereitung mit Aq. pro inj. liegt eine Suspension gasgefüllter, ca. 30 µm großer Kapseln vor.

### Beispiel 19

6 g Humanalbumin werden in 30 ml Aqua dest. gelöst und 3 Minuten mit einem Rotor-Stator-Dispergiergerät bei 20.000 Upm behandelt. Unter Weiterrühren werden 150 ml einer 2 % Span®85 enthaltenden Lösung von 1 Teil Chloroform und 4 Teilen Cyclohexan zugefügt. Nach 1 Minute werden 2 g Terephthalsäuredichlorid in 20 ml des organischen Lösungsmittels zugesetzt und nur noch mit einem Magnetrührer weitergerührt.

Nach 30 Minuten werden die flottierenden Kapseln abzentrifugiert und anschließend mit isotoner Kochsalzlösung gewaschen. Es ergibt sich eine Suspension etwa 30 µm großer, gasgefüllter Kapseln.

### Beispiel 20

200 ml 5 %ige Gelatinelösung von 60 °C und pH 4,5 werden 3 Minuten mit einem Rotor-Stator-Dispergiergerät bei 20.000 Upm behandelt. Unter ständigem Rühren werden 200 ml 5 %ige Gummi-Arabicum-Lösung zugefügt und der Ansatz auf 5 °C abgekühlt. Nach 2 Stunden werden 50 ml 3 %ige Glutaraldehydlösung zugefügt und der pH-Wert auf 8,5 eingestellt. Die flottierenden Kapseln mit einer Größe von 20-50 µm werden abzentrifugiert und mehrmals mit isotoner Kochsalzlösung, die 0,1 % Poloxamer 188 enthält, gewaschen.

### Beispiel 21

Eine Lösung aus 99,5 g Wasser und 0,5 g Poloxamer 407 wird mit 1 N Salzsäure auf pH 2,4 eingestellt. Zu dieser Lösung wird unter Rühren (4000 Upm) 1,0 g Cyanacrylsäurebutylester gegeben und 60 Minuten lang weitergerührt. Die erhaltene Suspension wird neutralisiert, die gasgefüllten Kapseln mit einer Größe von ca. 30-100 µm werden durch Zentrifugation abgetrennt und in einer zur Applikation geeigneten Flüssigkeit resuspendiert.

### Beispiel 22

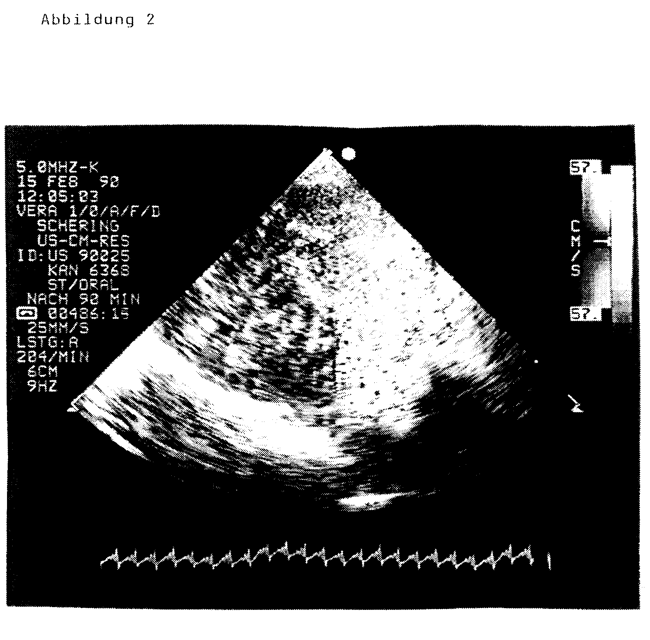

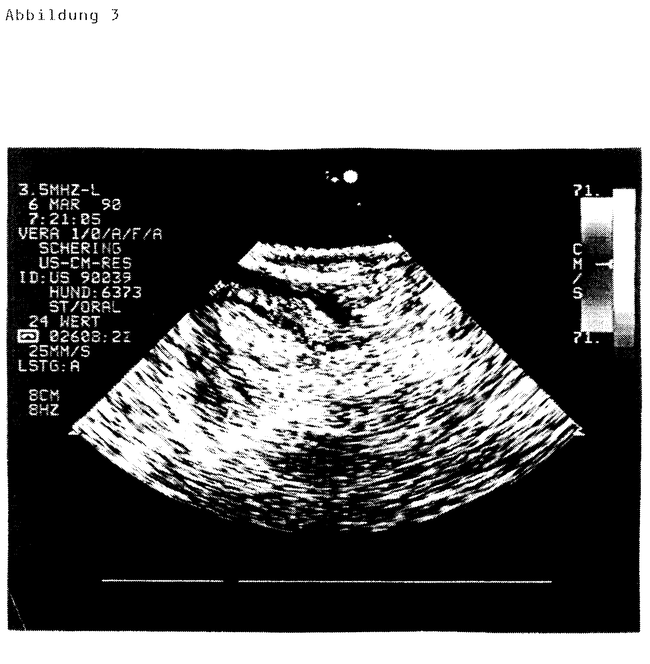

Einem 10 kg schweren narkotisierten Beagle-Hund werden über eine Schlundsonde 30 ml einer Suspension von gasgefüllten Mikropartikeln appliziert, die nach Beispiel 20 hergestellt wurden. Die Konzentration beträgt 1 mg/ml Suspension. Unmittelbar nach Applikation wird der Magen mit einem Farbdoppler-Ultraschallgerät untersucht. Das Lumen stellt sich farbig dar (Abb. 2). 30 Minuten später ist der Dünndarm ebenfalls markiert (Abb. 3).

### Beispiel 23

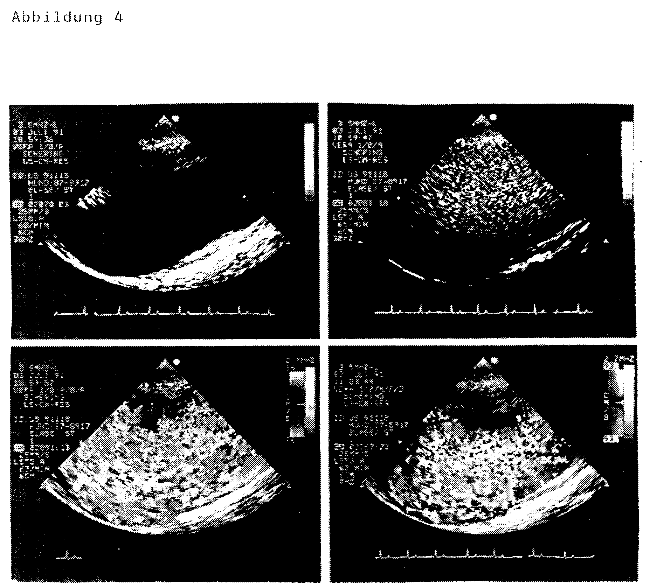

Einem in Narkose liegenden Beagle-Hund werden über einen Blasenkatheter in die flüssgkeitsgefüllte Harnblase 3 ml von nach Beispiel 19 hergestellten Mikrokapseln Konz. 10⁹ Partikel/ml appliziert. In Abb. 4 ist der Effekt vor und nach Kontrastmittelgabe im Farbdopplermode dargestellt.

## Patentansprüche

1. Verwendung von gas- oder flüssigkeitsgefüllten Mikrokapseln zur Herstellung eines Ultraschallkontrastmittels zur Kontrastierung von Körperregionen in denen im wesentlichen keine Bewegung der Mikrokapseln zu beobachten ist mittels Farbdoppler-Sonographie.

2. Verwendung von gas- oder flüssigkeitsgefüllten Mikrokapseln nach Anspruch 1 worin die gas- oder flüssigkeitsgefüllte Mikrokapseln aus Polycyanacrylaten oder biologisch abbaubaren Polyestern von α-, β- oder γ-Hydroxycarbonsäuren aufgebaut sind.

3. Verwendung von gas- oder flüssigkeitsgefüllten Mikrokapseln nach Anspruch 1 worin die gas- oder flüssigkeitsgefüllte Mikrokapseln aus biologisch abbaubaren Polymeren aufgebaut sind, die aus polymerisierbaren Aldehyden aufgebaut sind, die gewünschtenfalls zur Copolymerisation fähige Zusätze und/oder Crosslinker, gegebenenfalls Tenside oder Tensidgemische, Kopplungsagentien sowie gegebenenfalls über diese Kopplungsagenzienen gebundene Bio- oder Makromoleküle enthalten.

4. Verwendung von gas- oder flüssigkeitsgefüllten Mikrokapseln nach Anspruch 1 worin die gas- oder flüssigkeitsgefüllte Mikrokapseln aus Gelatine aufgebaut sind.

5. Verwendung von gas- oder flüssigkeitsgefüllten Mikrokapseln nach Anspruch 1 worin die gas- oder flüssigkeitsgefüllte Mikrokapseln aus teilweise denaturierten Proteinen aufgebaut sind.

6. Verwendung gemäß Anspruch 5 enthaltend als teilweise denaturiertes Protein denaturiertes Albumin oder denaturiertes humanes Serumalbumin.

7. Verwendung von gas- oder flüssigkeitsgefüllten Mikrokapseln gemäß den Ansprüchen 1 bis 6 zur Herstellung eines Kontrastmittels zur Kontrastierung des retikuloendothelialen Systems.

8. Verwendung von gas- oder flüssigkeitsgefüllten Mikrokapseln gemäß den Ansprüchen 1 bis 6 zur Herstellung eines Kontrastmittels zur Kontrastierung des Myocards.

9. Verwendung von gas- oder flüssigkeitsgefüllten Mikrokapseln gemäß den Ansprüchen 1 bis 6 zur Herstellung eines Kontrastmittels zur Kontrastierung des Gastrointestinaltrakts.

10. Verwendung von gas- oder flüssigkeitsgefüllten Mikrokapseln gemäß den Ansprüchen 1 bis 7 zur Herstellung eines Kontrastmittels zur Kontrastierung des Lymphsystems, der Leber oder der Milz.

## Claims

1. The use of gas- or liquid-filled microcapsules for the manufacture of an ultrasound contrast medium for contrasting by means of colour Doppler sonography regions of the body in which there is substantially no observable movement of the microcapsules.

2. The use of gas- or liquid-filled microcapsules according to claim 1, wherein the gas- or liquid-filled microcapsules are synthesised from polycyanoacrylates or biologically degradable polyesters of α-, β- or γ-hydroxycarboxylic acids.

3. The use of gas- or liquid-filled microcapsules according to claim 1, wherein the gas- or liquid-filled microcapsules are synthesised from biologically degradable polymers synthesised from polymerisable aldehydes which, if desired, contain additives capable of copolymerisation and/or crosslinkers, optionally surfactants or surfactant mixtures, coupling agents and optionally bio- or macro-molecules bonded by way of those coupling agents.

4. The use of gas- or liquid-filled microcapsules according to claim 1, wherein the gas- or liquid-filled microcapsules are synthesised from gelatin.

5. The use of gas- or liquid-filled microcapsules according to claim 1, wherein the gas- or liquid-filled microcapsules are synthesised from partially denatured proteins.

6. The use according to claim 5 comprising, as partially denatured protein, denatured albumin or denatured human serum albumin.

7. The use of gas- or liquid-filled microcapsules according to claims 1 to 6 for the manufacture of a contrast medium for contrasting the reticuloendothelial system.

8. The use of gas- or liquid-filled microcapsules according to claims 1 to 6 for the manufacture of a contrast medium for contrasting the myocardium.

9. The use of gas- or liquid-filled microcapsules according to claims 1 to 6 for the manufacture of a contrast medium for contrasting the gastrointestinal tract.

10. The use of gas- or liquid-filled microcapsules according to claims 1 to 7 for the manufacture of a contrast medium for contrasting the lymphatic system, the liver or the spleen.

## Revendications

1. Utilisation de microcapsules remplies de gaz ou de liquide pour la fabrication d'un produit de contraste aux ultrasons, pour contraster des régions du corps dans lesquelles sensiblement aucun mouvement des microcapsules n'est à observer, au moyen d'une sonographie Doppler en couleurs.

2. Utilisation de microcapsules remplies de gaz ou de liquide suivant la revendication 1, caractérisée en ce que les microcapsules remplies de gaz ou de liquide sont constituées de polycyanacrylates ou de polyesters biologiquement dégradables d'acides hydroxycarboxyliques α, β ou γ.

3. Utilisation de microcapsules remplies de gaz ou de liquide suivant la revendication 1, caractérisée en ce que les microcapsules remplies de gaz ou de liquide sont édifiées à partir de polymères biologiquement dégradables qui sont constitués à partir d'aldéhydes polymérisables qui contiennent éventuellement des additifs susceptibles de copolymérisation et/ou des agents de réticulation, éventuellement des agents tensioactifs ou des mélanges de tensioactifs, des agents de couplage ainsi qu'éventuellement des biomolécules ou macromolécules liées par l'intermédiaire de ces agents de couplage.

4. Utilisation de microcapsules remplies de gaz ou de liquide suivant la revendication 1, caractérisée en ce que les microcapsules remplies de gaz ou de liquide sont édifiées à partir de gélatine.

5. Utilisation de microcapsules remplies de gaz ou de liquide suivant la revendication 1, caractérisée en ce que les microcapsules remplies de gaz ou de liquide sont édifiées à partir de protéines partiellement dénaturées.

6. Utilisation suivant la revendication 5, contenant, comme protéines partiellement dénaturées, de l'albumine dénaturée ou de l'albumine de sérum humain dénaturée.

7. Utilisation de microcapsules remplies de gaz ou de liquide suivant l'une des revendications 1 à 6, pour la fabrication d'un produit de contraste destiné à contraster le système réticulo-endothélial.

8. Utilisation de microcapsules remplies de gaz ou de liquide suivant l'une des revendications 1 à 6, pour la fabrication d'un produit de contraste destiné à contraster le myocarde.

9. Utilisation de microcapsules remplies de gaz ou de liquide suivant l'une des revendications 1 à 6, pour la fabrication d'un produit de contraste destiné à contraster le tractus gastro-intestinal.

10. Utilisation de microcapsules remplies de gaz ou de liquide suivant l'une des revendications 1 à 7, pour la fabrication d'un produit de contraste destiné à contraster le système lymphatique, le foie ou la rate.
